# EUROPEAN PATENT SPECIFICATION

(11) **EP 4 661 761 B1**
(45) Date of publication and mention of the grant of the patent: **29.07.2026**
(21) Application number: 24702546.3
(22) Date of filing: 30.01.2024
(51) Int. Cl.: A61B 6/04, A61B 5/107, A61B 5/00

(54) **THREE-DIMENSIONAL IMAGING OF A SUBJECT ON A SUBJECT SUPPORT**
DREIDIMENSIONALE BILDGEBUNG EINER PERSON AUF EINER PATIENTENLIEGE
IMAGERIE TRIDIMENSIONNELLE D'UN SUJET SUR UN SUPPORT DE SUJET

(30) Priority: 06.02.2023 EP 23155024
(43) Date of publication of application: 17.12.2025
(73) Proprietor: Koninklijke Philips N.V., 5656 AE Eindhoven (NL)
(72) Inventor: KRUEGER, Sascha, 5656 AG Eindhoven (NL); SOMMER, Karsten, 5656 AG Eindhoven (NL); FRERKING, Lena Christina, 5656 AG Eindhoven (NL)
(74) Representative: Philips Intellectual Property & Standards
(86) International application number: PCT/EP2024/052165
(87) International publication number: WO 2024/165375

(56) References cited:
- US-A1- 2009 285 357
- US-A1- 2019 394 449
- US-A1- 2021 104 055
- "16th European Conference - Computer Vision - ECCV 2020", vol. 8673, 1 January 2014, CORNELL UNIVERSITY LIBRARY,, 201 Olin Library Cornell University Ithaca, NY 14853, article VIVEK SINGH ET AL: "Estimating a Patient Surface Model for Optimizing the Medical Scanning Workflow : 17th International Conference, Boston, MA, USA, September 14-18, 2014, Proceedings, Part I", pages: 472 - 479, XP055737512, DOI: 10.1007/978-3-319-10404-1_59

## Description

### FIELD OF THE INVENTION

The invention relates to medical imaging, in particular to the configuration of a medical imaging system.

### BACKGROUND OF THE INVENTION

Various tomographic medical imaging techniques such as Magnetic Resonance Imaging (MRI), Computed Tomography, Positron Emission Tomography, and Single Photon Emission Tomography enable detailed visualization of anatomical structure of a subject. Often times it can be beneficial to determine the position of a subject before the subject is inserted into a medical imaging system.

United States patent application publication US2022287669A1 discloses an automatic light arrangement for medical visualization includes: providing a medical 3D image, providing spatial information about a region of interest in this 3D-image and spatial information about a virtual camera, determining a plurality of possible arrangements for light sources by using depth information based on the 3D image together with the spatial information about the region of interest in this image and the spatial information about the virtual camera, wherein valid arrangements are those where shadows on the region of interest are below a predefined threshold, and/or wherein the determination or arrangements is based on a number of predefined perceptual metrics applied specifically to the regions of interest, prioritizing the determined arrangements, and choosing the arrangement with the best prioritization.

United States patent application publication US2009/285357A1 discloses a 3D optical system to obtain optical and depth images of a patient while the patient is moved into the bore to obtain a whole body 3D mesh used to identify various body sections of the patient.

United States patent application publication US2019/394449A1 discloses a plastic surgery planning system using double optics to obtain a 3D image and distance information of a stationary patient.

United States patent application publication US2021/104055A1 discloses a system to check a patient's position on a table in preparation of a medical scan procedure using a 3D image obtained from a capture device.

### SUMMARY OF THE INVENTION

The invention is set out in the appended set of claims. The invention provides for a medical system, a method and a computer program in the independent claims. The embodiments are given in the dependent claims.

A difficulty in using stereo cameras for imaging subjects on a subject support is that a pair of stereo cameras are typically not able to image the entire subject. There may therefore be obscured regions in the three-dimensional image. Embodiments may provide for an improved means of providing a composite three-dimensional image. A first camera and a second camera are used to image a subject on a subject support that can move along a predetermined path between a first position, at least one intermediate position, and a second position. **In** the first position, the first camera acquires initial first camera image data and the second camera acquires initial second camera image data. **In** one of the intermediate positions the first camera acquires additional first camera image data and the second camera acquires additional second camera image data. An initial three-dimensional image is reconstructed from the initial first camera data and the initial second camera data.

Since the displacement along the predetermined path between the first position and the intermediate position is known, this information is used to reconstruct a first camera three-dimensional image from the initial first camera image data and the additional first camera image data. This information is further used to reconstruct a second camera three-dimensional image from the initial second camera image data and the additional second camera image data. A composite three-dimensional image is then constructed by combining the initial three-dimensional image, the first camera three-dimensional image and the second camera three-dimensional image.

**In** one aspect the invention provides for a medical system that comprises a subject support. The subject support comprises a support surface for receiving a subject. The medical system further comprises a first camera configured for imaging a first portion of the support surface. The medical system further comprises a second camera which is configured for imaging a second portion of the support surface. The first portion and the second portion comprise an overlapping region. The subject support is moveable relative to the first camera and the second camera along a predetermined path between a first position, at least one intermediate position, and a second position. Each of the one or more intermediate positions is located between the first position and the second position.

The medical system further comprises a memory containing machine-executable instructions. The medical system further comprises a computational system that is configured for controlling the medical system. For example, it may be used for controlling the first camera, the second camera and the subject support. Execution of the machine-executable instructions causes the computational system to control the first camera to acquire initial first camera image data descriptive of a subject on the subject support when the subject support is in the first position. Execution of the machine-executable instructions further causes the computational system to control the second camera to acquire initial second camera image data that is descriptive of the subject on the subject support when the subject support is in the first position. Execution of the machine-executable instructions further causes the computational system to construct an initial three-dimensional image of the subject using at least a portion of the overlapping region of the initial first camera image data and the initial second camera image data.

Execution of the machine-executable instructions further causes the computational system to identify as a first obstructed region a region of the subject imaged by the initial first camera image data but obstructed in the initial second camera image data and to identify as a second obstructed region a region of the subject imaged by the initial second camera image data but obstructed in the initial first camera image data. So in the previous steps an image was acquired with the first camera and the second camera. This was then used to construct an initial three-dimensional image.

Execution of the machine-executable instructions further causes the computational system to perform at least once the controlling of the subject support to move to one of the one or more intermediate positions. Execution of the machine-executable instructions further causes the computational system to perform at least once to control the first camera to acquire additional first camera image data that is descriptive of the subject on the subject support when the subject support is in the one of the one or more intermediate positions.

Execution of the machine-executable instructions further causes the computational system to perform the following at least once and this is to control the second camera to acquire additional second camera image data that is descriptive of the subject on the subject support when the subject support is in the one of the one or more intermediate positions. Execution of the machine-executable instructions further causes the computational system to construct a first camera three-dimensional image of the subject using the initial first camera image data and the additional first camera image data. The image of the subject has been taken in at least two positions using the first camera. The images from this first camera alone are used to construct the first camera three-dimensional image.. A potential benefit may be that one could possibly be able to select the one intermediate other position where for a given (unknown) object, the reconstruction has smallest obstructed areas, or only in areas of less interest, or minimal obstructions in area of interest. This may likely be the case because obstructed areas are often different than those occurring for the two camera at one position.

Execution of the machine-executable instructions further causes the computational system to construct a second camera three-dimensional image of the subject using the initial second camera image data and the additional second camera image data. Likewise, a potential benefit is that the multiple images acquired by the second camera are less likely to that will be obstructed regions in the second camera three-dimensional image.

Execution of the machine-executable instructions further causes the computational system to construct a composite three-dimensional image, the first camera three-dimensional image, and the second camera three-dimensional image. The first obstructed region is at least partially replaced by the first camera three-dimensional image. The second obstructed region is at least partially replaced by the second camera three-dimensional image. When the subject was in the first position the images from the first camera and the second camera were used to construct the initial three-dimensional image. By moving to the intermediate position and taking additional images, the first camera three-dimensional image and the second camera three-dimensional image were also able to be constructed.

The medical system may also have a calibration within the memory in some examples. For example, a graph paper or other object which has a discernible pattern may be placed on the support surface to perform a calibration. The support may then be moved from the first position to one or more of the intermediate positions and images acquired. This can be used to make a mapping of how the surface moves from the initial image to the intermediate images as a function of the subject support. If there is a subject or other object on top of the support surface, then those portions of the object or subject are closer to the camera than the support surface will be. This may have the effect of moving a larger distance between images than for something which is directly on the support surface and doesn't have a height.

As an alternative to having a calibration in the memory, the extrinsic parameters of the camera with respect to the medical apparatus are known, i.e. the 3x3 rotation matrix and 1x3 translation vector describing the angulations and position of the camera with respect to the reference coordinate system of the medical apparatus.

This may allow transforming the coordinates of a pixel in the camera image into 3D coordinates of the corresponding point in the coordinate system of the medical apparatus.

As a further alternative, if the camera is not calibrated with respect to the medical apparatus, a height profile of the subject can still be derived by processing the camera images alone, however in this case the absolute position would not be known. However, if the imaging system or rigid parts thereof is partly in the FOV of the first or second cameras, auto-registration can be used to derive the registration.

In another embodiment, the predetermined path is a linear path.

In another embodiment, the predetermined path is a linear path where the subject support may be raised or lowered a predetermined amount while it traverses the linear path.

In another embodiment, the first camera and the second camera may have portions of the subject which are obstructed in their respective images. This may have the benefit of providing for a complete three-dimensional image of the subject the subject support is moved to at least one of the intermediate positions.

In another embodiment, the images used to construct the composite three-dimensional image are pieced together to provide a more complete three-dimensional image of the subject. In some cases, portions of the images may be connected together. If regions of the subject of these various images overlap one option would be to calculate average values of the image thereby reducing the amount of noise in the composite three-dimensional image.

In another embodiment execution of the machine-executable instructions further causes the computational system to additionally perform at least once the construction of an additional three-dimensional image of the subject using additional first camera image data and the additional second camera image data. Execution of the machine-executable instructions further causes the computational system to additionally use the additional three-dimensional image when the composite three-dimensional image is constructed. This may for example be useful in averaging with the other three-dimensional images when constructing the composite three-dimensional image. This may have an effect of reducing the amount of noise in the composite three-dimensional image and it may for example provide for a more accurate means of constructing a composite three-dimensional image of a subject.

In another embodiment the composite three-dimensional image comprises average regions constructed by averaging overlapping regions between the two or more of the following: the initial three-dimensional image, the first camera three-dimensional image, the second camera three-dimensional image, and the additional three-dimensional image, and previous iterations of the composite three-dimensional image. For example, as the subject is moved to multiple intermediate positions there may be additional images that are available and which can be averaged into the existing composite three-dimensional image. This may provide for a means of producing a very high resolution or a more accurate composite three-dimensional image of the subject.

In another embodiment the memory further comprises an anatomical key point module configured to output a set of anatomical key points for the subject in response to receiving any one of the following: the initial first camera image data, the initial second camera image data, the additional first camera image data, and the additional second camera image data. Execution of the machine-executable instructions further causes the computational system to receive initial first camera key point data in response to inputting the initial first camera image data into the anatomical key point module and to receive initial camera key point data in response to inputting the initial second camera image data into the anatomical key point module.

This embodiment may be beneficial because there are excellent algorithms available for identifying anatomical key points in subjects. By identifying in both of the two cameras, the first camera and the second camera, a stereo location or three-dimensional location of the anatomical key points can be determined. This may be particularly beneficial because it may provide for an improved means of aligning the various three-dimensional images to produce the composite three-dimensional image as well as providing for a means of detecting subject motion or other problems during the process of acquiring various images and constructing the composite three-dimensional image.

An anatomical key point as used herein encompasses an anatomical landmark or position located in the subject. Anatomical key points could incorporate the locations of various joints or surface landmarks (eyes, ears, nose) of the subject. The camera image is descriptive of a subject and provides a description of the exterior or surface of the subject (texture and/or shape). For example, the camera image could be an optical image, an infrared image, thermal, or even a color image. In other examples the camera image is a three-dimensional surface image. In another example, the camera image is a composite image formed from multiple camera images. For example, two two-dimensional images could be used to provide a stereo image which provides three-dimensional or spatial information. In other examples the camera image is a composite image formed from multiple types of images. An infrared or thermal image could be combined with a three-dimensional image.

**In** another embodiment the anatomical key point module comprises a neural network configured to output a separate anatomical key point coordinate probability map for each of the at least one anatomical key point coordinates in response to receiving the image of the subject on the subject support. For example, various joints and locations within the subject may be included in the anatomical key point coordinates. For each of the set there may be a separate image or map which is output and the probability of the joint or anatomical location being in a particular position is output on this map. The most likely position can for example be obtained by taking the maximum probability. This system may be particularly good at identifying the location of joints or other anatomical locations when they are obscured or partially obscured. For example, if the subject has clothing or a blanket placed on the subject.

Execution of the machine-executable instructions further causes the computational system to receive the separate anatomical key point coordinate probability map in response to inputting the image into the neural network. Execution of the machine-executable instructions further causes the computational system to calculate the set of anatomical key point coordinates from the separate joint coordinate probability map for each of the set of joint coordinates.

The training of the neural network may be achieved by having images of the subject and then having training key point coordinate probability maps for each of the at least one anatomical key point coordinates that are labeled. For example, one could have a series of images of different subjects in slightly different positions wearing different clothing or even being covered with blankets or having the body partially obscured. An operator could then go and mark up various points so that the locations of the anatomical key point coordinates are indicated. This can then be used for example in a deep learning algorithm to train the neural network.

In another embodiment execution of the machine-executable instructions further causes the computational system to calculate the one of the one or more intermediate positions using a location of anatomical key points of the first camera key point data and/or using the location of anatomical key points of the initial second camera key point data. For example, the location of the first obstructed region could be referenced using the initial first camera key point data and the location of the second obstructed region could be located using the initial second camera key point data. These key points are located in their respective images and as a result the location of the obstructed regions in the image can be inferred. Once the location of these obstructed regions is known, the intermediate position can then be determined to obtain a suitable position for acquiring the additional first camera image data and the additional second camera image data.

In another embodiment, execution of the machine-executable instructions further causes the computational system to receive image-guided therapy position data descriptive of predetermined anatomical key points of the subject relative to the subject support. Execution of the machine-executable instructions further causes the computational system to detect a subject positioning error by comparing the predetermined anatomical key points to the initial first camera key point data and/or the initial second camera key point data.

Execution of the machine-executable instructions further causes the computational system to provide a warning signal if the subject positioning error is detected. For example, the warning signal might cause a user interface, audio system, or other human interface device to provide a warning signal. **In** other cases it may be used to disable a radiotherapy device if the subject has moved too much. **In** this embodiment the position of the subject on the subject support is compared to the data or key points that are acquired as the subject is placed on the subject support. This may help to prevent an error in the positioning of the subject during a radiotherapy procedure. Without the use of the anatomical key points subject motion may be difficult to detect with a camera system because there are other moving parts or objects in the images as well as shadows which can obscure the position of a subject.

**In** another embodiment execution of the machine-executable instructions further causes the computational system to receive additional first camera key point data in response to inputting the additional first camera image data into the anatomical key point module. Execution of the machine-executable instructions further causes the computational system to receive additional second camera key point data in response to inputting the additional second camera image data into the anatomical key point module. Execution of the machine-executable instructions further causes the computational system to detect a subject motion condition between the first position of the subject support and the one of the one or more intermediate positions by detecting a difference between the initial first camera key point data and the additional first camera key point data and/or a difference between the initial second camera key point data and the additional second camera key point data.

The placement of the subject support is known so a simple translation can be used to compare coordinates between the initial first camera key point data and the second additional first camera key point data or the difference between the initial second camera key point data and the additional second camera key point data. Execution of the machine-executable instructions further causes the computational system to exclude at least a portion of the first camera three-dimensional image and/or the second camera three-dimensional image from the composite three-dimensional image if the subject's motion condition is detected. In one instance, if the subject has moved, then particular three-dimensional images or image data could be thrown out or excluded. In other cases, for instance if the subject moved just a hand or a foot, not necessarily all of the images need to be thrown out. For example, if a particular key point moves a neighborhood around this key point could be identified and image data in the vicinity of this key point could be excluded from construction of the composite three-dimensional image of the subject.

In another embodiment the subject support has a maximum width that is perpendicular to the predetermined path. The stereo baseline is between 10% and 200% of the maximum width of the subject support. This embodiment may be beneficial because it may provide for an effective means of positioning the first camera and the second camera to obtain high-quality three-dimensional images.

In another embodiment the stereo baseline is preferably between 50% and 150% of the maximum width of the subject support. Choosing the stereo baseline to having a width of between 50% and 150% may provide for an effective means of eliminating the occluded regions.

In another embodiment execution of the machine-executable instructions further causes the computational system to identify at least one stationary object region by detecting stationary voxels between the initial first camera image data and the additional first camera image data and thereby detecting stationary voxels between the initial second camera image data and the additional second camera image data. The at least one stationary object region is excluded from the composite three-dimensional image of the subject. The subject support is moved between the first position and the intermediate positions. If an object remains stationary in images acquired when the subject support is in different positions, this means that the object is in fact stationary. Such objects may be excluded because they are for example not part of the subject or objects being moved by the subject support.

In another embodiment the medical system further comprises a medical imaging system for acquiring measurement data from an imaging volume. The medical imaging system may for example be a tomographic medical imaging system. At least a portion of the support surface is within the imaging volume when the subject support is within the second position. Execution of the machine-executable instructions further causes the computational system to control the subject support to move to the second position. Execution of the machine-executable instructions further causes the computational system to control the medical imaging system to acquire the measurement data.

In another embodiment execution of the machine-executable instructions further causes the computational system to reconstruct a medical image from the measurement data.

In another embodiment execution of the machine-executable instructions further causes the computational system to use the composite three-dimensional image of the subject to calculate the scanning isocenter.

In another embodiment execution of the machine-executable instructions further causes the computational system to use the composite three-dimensional image for subject positioning and orientation.

In another embodiment execution of the machine-executable instructions further causes the computational system to use the composite three-dimensional image to perform collision risk prediction with the medical imaging system.

In another embodiment if the medical system is a magnetic resonance imaging system the computational system uses the composite three-dimensional image to calculate an SAR estimate using the subject model.

In another embodiment execution of the machine-executable instructions further causes the computational system to use the composite three-dimensional image of the subject to calculate a subject weight.

In another embodiment execution of the machine-executable instructions further causes the computational system to calculate a subject height using the composite three-dimensional image of the subject.

In another embodiment the medical imaging system is a magnetic resonance imaging system.

In another embodiment the medical imaging system is a positron emission tomography system.

In another embodiment the medical imaging system is a single-photon emission tomography system.

In another embodiment the medical imaging system is a computed tomography system.

In another embodiment the medical imaging system is a combined positron emission tomography and magnetic resonance imaging system.

In another embodiment the medical imaging system is a combined positron emission tomography and computed tomography system.

In another embodiment the medical imaging system is a combined computed tomography and radiation therapy system.

In another embodiment the medical imaging system is a combined computed tomography and positron emission tomography system.

In another embodiment the medical imaging system is a combined magnetic resonance imaging system and a radiation therapy system.

In another embodiment the medical imaging system is part of an image-guided radiation therapy system.

In another aspect the invention provides for a method of controlling a medical system. The medical system comprises a subject support. The subject support comprises a support surface for receiving a subject. The medical system further comprises a first camera configured for imaging a first portion of the support surface. The medical system further comprises a second camera configured for imaging a second portion of the support surface. The first portion and the second portion comprise an overlapping region. The subject support is moveable relative to the first camera and the second camera along a predetermined path between a first position, at least one intermediate position, and a second position. Each of the one or more intermediate positions is located between the first position and the second position.

The method comprises controlling the first camera to acquire initial first camera image data descriptive of a subject on the subject support when the subject support is in the first position. The method further comprises controlling the second camera to acquire initial second camera image data descriptive of the subject on the subject support when the subject support is in the first position. The method further comprises constructing an initial three-dimensional image of the subject using at least a portion of the overlapping region of the initial first camera image data and the initial second camera image data.

The method further comprises identifying a first obstructed region of the subject imaged by the initial first camera image data but obstructed in the initial second camera image data. The method further comprises identifying as a second obstructed region a region of the subject imaged by the initial second camera image data but obstructed in the initial first camera image data. Execution of the machine-executable instructions further causes the computational system to perform the following at least once. This includes controlling the subject support to move to the one or more intermediate positions. This also includes controlling the first camera to acquire additional first camera image data that is descriptive of the subject on the subject support when the subject support is in the one of the one or more intermediate positions. This also includes controlling the second camera to acquire additional second camera image data descriptive of the subject on the subject support when the subject support is in the one of the one or more intermediate positions. This also includes constructing a first camera three-dimensional image of the subject using the initial first camera image data and the additional first camera image data. This also includes constructing a second camera three-dimensional image of the subject using the initial second camera image data and the additional second camera image data. And finally, this also includes constructing a composite three-dimensional image of the subject using the initial three-dimensional image, the first camera three-dimensional image, and the second camera three-dimensional image. The first obstructed region is at least partially replaced by the first camera three-dimensional image. The second obstructed region is at least partially replaced by the second camera three-dimensional image.

In another aspect the invention provides for a computer program comprising machine-executable instructions for execution by a computational system that controls a medical system. The computer program may for example be stored on a non-transitory storage medium. The medical system comprises a subject support. The subject support comprises a support surface for receiving a subject. The medical system further comprises a first camera that is configured for imaging a first portion of the support surface. The medical system further comprises a second camera that is configured for imaging a second portion of the support surface. The first portion and the second portion comprise an overlapping region. The subject support is moveable relative to the first camera and the second camera along a predetermined path between a first position, at least one intermediate position, and a second position. Each of the one or more intermediate positions is located between the first position and the second position.

Execution of the machine-executable instructions causes the computational system to control the first camera to acquire initial first camera image data descriptive of a subject on the subject support when the subject support is in the first position. Execution of the machine-executable instructions further causes the computational system to control the second camera to acquire initial second camera image data descriptive of the subject on the subject support when the subject support is in the first position. Execution of the machine-executable instructions further causes the computational system to construct an initial three-dimensional image of the subject using at least a portion of the overlapping region of the initial first camera image data and the initial second camera image data. Execution of the machine-executable instructions further causes the computational system to identify as a first obstructed region a region of the subject imaged by the initial first camera image data but obstructed in the initial second camera image data.

Execution of the machine-executable instructions further causes the computational system to identify as a second obstructed region a region of the subject imaged by the initial second camera image data but obscured in the initial first camera image data. Execution of the machine-executable instructions further causes the computational system to perform the following at least once, which includes to control the subject support to move to the one or more intermediate positions. This further comprises controlling the first camera to acquire additional first camera image data that is descriptive of the subject on the subject support when the subject support is in one of the one or more intermediate positions. This also includes controlling the second camera to acquire additional second camera image data that is descriptive of the subject on the subject support when the subject support is in the one of the one or more intermediate positions. This also includes constructing a first camera three-dimensional image of the subject using the initial first camera image data and the additional first camera image data. This further includes constructing a second camera three-dimensional image of the subject using the initial second camera image data and the additional second camera image data. This further includes constructing a composite three-dimensional image of the subject using the initial three-dimensional image, the first camera three-dimensional image, and the second camera three-dimensional image. The at least first obstructed region is at least partially replaced by the first camera three-dimensional image. The second obstructed region is at least partially replaced by the second camera three-dimensional image.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Measurement data is defined herein as being recorded measurements made by a tomographic medical imaging system descriptive of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of measurement data.

A Magnetic Resonance Imaging (MRI) image or MR image is defined herein as being the reconstructed two-, three-, or four-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 shows a two-dimensional rendering of a three-dimensional image;
Fig. 2 illustrates an example of a medical system;
Fig. 3 illustrates a further example of a medical system;
Fig. 4 shows a flow chart which illustrates a method of using the medical system of Fig. 3;
Fig. 5 illustrates a further example of a medical system;
Fig. 6 illustrates a further example of a medical system; and
Fig. 7 shows a flow chart which illustrates a method of using the medical system of Fig. 6.

### DESCRIPTION OF EMBODIMENTS

Like numbered elements in these Figs are either equivalent elements or perform the same function. Elements which have been discussed previously will not necessarily be discussed in later Figs if the function is equivalent.

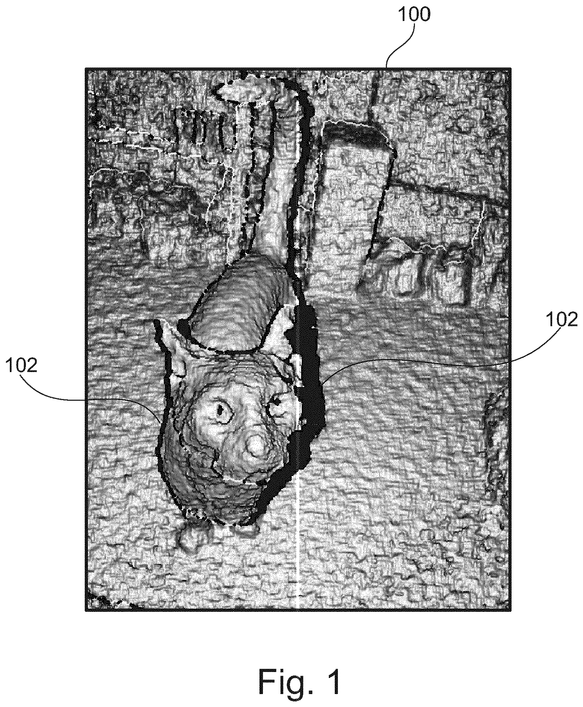

Stereo-based depth-sensing including structured-light enhancement techniques suffer from shadowing in areas where not both cameras have line of sight. Typically, these problem areas occur at the edges of objects. This effect is illustrated in Fig. 1 below.

Fig. 1 shows an example two-dimensional rendering of a three-dimensional image. This image shows a three-dimensional image of a cat. On either side of the cat there are two black bands visible 102. These black bands are obstructed regions 102. The cause of these obstructed regions 102 is that in this region it is not visible to both cameras. For this reason, within the obstructed regions 102 it is not possible to construct the three-dimensional image 100. These are essentially holes in the three-dimensional image.

To overcome this, recently even AI -based algorithms have been proposed to fill the resulting data gaps with reasonable values. The downside of this approach is that those values are not actually measured and therefore can be erroneous. A similar issue arises in outside areas where the FOVs of the two cameras have no overlap. In this case there is no stereo data at all which can be matched from one camera to the other. The effect is that the FOV is reduced with respect to the native FOV of each camera of the stereo pair.

These commonly known shortcomings of stereovision are hard to solve in general for robotic navigation purposes, but can be overcome in the context of medical imaging examinations, which involve patient supports that can be automatically moved in a controlled way along defined axes. Thus, no additional sensors are needed to fill the data gaps with real data. Instead, the table motion axis is used to obtain additional images the two cameras, respectively. Using these additional images together with the known table displacement between the images acquisitions allows to fill the data gaps and to produce a dense depth map with same resolution as the intrinsic resolution of the cameras. One main insight and trick here is that any two suitable timepoints are chosen from [tStart,tEnd] of any table travel event. Such a table travel event is a phase where the patient is immobilized or stabilized, and the exam setup is not changed. Therefore, the depth errors resulting from motion is minimal. Several such events may exist during an exam preparation, at least one, which is the table insertion from the out-down to up-in position of the table.

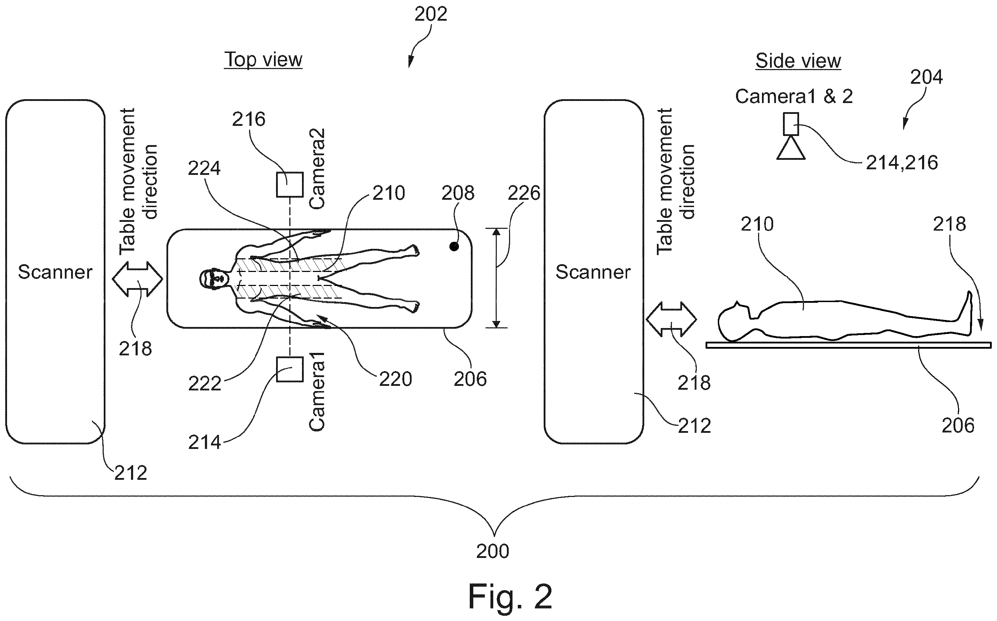

Fig. 2 illustrates a simplified illustration of a medical system 200. Not all components of the medical system 200 are shown in Fig. 2. A top view 202 and a side view 204 are shown adjacent to each other. The medical system 200 is shown as comprising a subject support 206 with a support surface 208. A subject 210 is residing on the support surface 208. The subject support 206 is shown in a first position and can be moved in a predetermined path 218 to position the subject 210 within a medical imaging system 212. There is a first camera 214 and a second camera 216 positioned above the support surface 208 so that it is able to image the top of the subject 210 and portions of the support surface 208 that are exposed. There is a baseline 220 between the two cameras 214, 216 that is perpendicular to the predetermined path. On the subject 210 a first obstructed region 222, which is a region that can be imaged by camera one but not camera two 216 there is also a second obstructed region 224 which can be imaged by camera two 216 but not camera one 214. The subject support 206 has a width 226. The width of the baseline 220 may be defined in terms of its width relative to that of the subject support 226. In the side view 204, The view of camera one 214 and camera two 216 are overlapping.

It can be seen that when the subject support 206 is in the first position the obstructed regions 222 and 224 are not able to be made into a three-dimensional image. However, if the subject support 206 is moved along the predetermined path 218 and multiple images are acquired then these multiple images from either camera 1, 214 or camera 2, 216 may be used to construct additional three-dimensional images that can be used to fill in the obstructed regions 222, 224

The two cameras 214, 216 are connected to a computational system which may be connected to the MR scanning (or other imaging modality) hardware and software. The computational system receives the table position and the camera images. The cameras 214, 216 also receives the table motion state or position and may selects an arbitrary number of camera images for a given table travel event in the time interval [tStart, tEnd]. The disparity between pairs of images during this event is computed. The image frames can be adjacent or at arbitrary timepoints during the table travel. A neural network can optionally be used to determine suitable frame candidates where optical flow in the image is limited to that of coherent table motion. For example software for determining the location of anatomical key points can be used for this determination.

The travelled distance between any two acquired images may be used to convert the calculated disparity into depth maps. Stationary parts in the scene have the most robust stereo matching performance and result in infinite distance. These regions are filtered out. Additionally, the table region may be calibrated and the disparity calculation can be limited to the table area. Multiple image frames during table travel can be used to identify non-coherent motion or static obstructions. Disparity information from multiple image pairs can be used to compose a dense disparity map by removing spurious non-coherent motion areas or static obstructions and filling the information with dependable values from other image pairs if needed. When connecting this technology to a patient key point detection AI network, this gap filling can be concentrated on the examination target area or a specific part of the examination setup. In addition, the patient key point detection network can be used to detect patient motion events during table travel, e.g., by comparing relative key point locations between the chosen camera images. This can be useful to ensure that patient motion does not corrupt results of the proposed algorithm.

One fundamental advantage of this over stereo matching with a static baseline is that multiple baseline measurements are available for each image patch. This allows to cross validate confidence values of the stereo matching, reduce noise with averaging or to select the most reliable measurement from the series of images. The arbitrary number of matching computations can be elegantly parallelized using current GPU architectures.

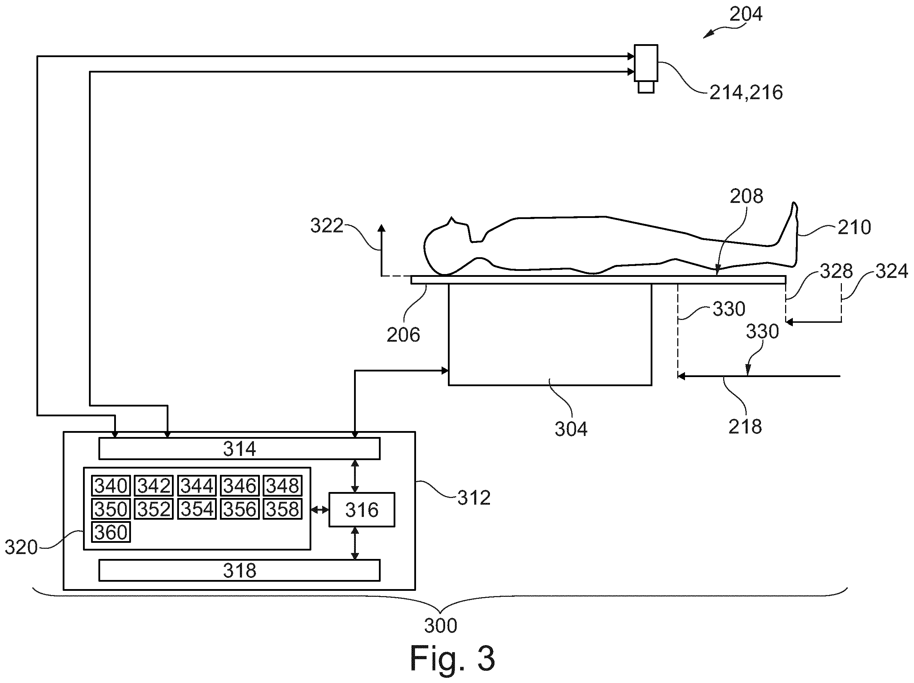

Fig. 3 illustrates an example of a medical apparatus 300. The medical apparatus is shown as comprising a subject support 206. The subject support 206 comprises an actuator 304 which is constructed to move the subject support 206 a controlled distance or displacement along a predetermined path 218. There is a subject 210 reposing on a support surface 208. The support surface 208 is facing two camera 214, 216. A side view 204 is shown, so the view of the two cameras 214, 216 overlap. The cameras 214, 216 is able to acquire images of the support surface 208 and/or the subject 210 when the subject support 206 is in various positions. The medical apparatus 300 is further shown as comprising a computer 312. The computer 312 comprises a hardware interface 314 that enables a computational system 316 to communicate with and control the other components of the medical apparatus 300.

Specifically in this Fig. the hardware interface 314 is shown as interfacing with the two camera 314, 316 to acquire images and with the actuator 304 to control the position of the subject support 206. In other examples or embodiments the hardware interface 314 may be used to control additional components such as a medical imaging system 212 (or scanner).

The computational system 316 is in communication with the hardware interface 314, a memory 320, and an optional user interface 318. The memory 320 may be any combination of memory which is accessible to the computational system 316. This may include such things as main memory, cached memory, and also non-volatile memory such as flash RAM, hard drives, or other storage devices. **In** some examples the memory 320 may be considered to be a non-transitory computer-readable medium.

The arrow 322 indicates a height or distance above the support surface 208. The dashed line 324 indicates the location of an edge of the subject support 206 when the subject support is in the first position 324. The dashed line 330 indicates the position of the edge of the subject support 206 when the subject support 206 is in the second position 330. The dashed line 328 shows the current position of the edge of the subject support 206. The subject support 206 is currently in an intermediate position 328. As the subject support 206 is moved to different displacements 332, 334 with respect to the first position 324, images are acquired with the cameras 214, 216. Portions of the subject 210 that are closer to the cameras 214, 216 than the support surface 208 may move larger within the multiple images than the support surface 208.

For example, a graph or other pattern could be placed on the support surface 208 and the support surface could be imaged in multiple positions. This may provide information on how the displacements 332, 334 relate to pixel displacement of images of the support surface 208. When a subject 210 is placed on the support surface 208 the pixels which represent identical portions of the subject 210 will move a larger amount than what would move if the support surface 208 alone were moved. This larger movement of individual pixels or groups of pixels may be used to develop a three-dimensional measurement of the distance of the surface of the subject 210 above 322 the support surface 208.

The memory 320 is shown as containing machine-executable instructions 340. The machine-executable instructions enable the computational system 316 to perform various computational tasks such as calculating the three-dimensional images from several two-dimensional images. The memory 320 is further shown as containing the initial first camera image data 342 and the initial second camera image data 344. The memory 320 is further shown as containing the initial three-dimensional image 346 that was calculated from the initial first camera image data 342 and the initial second camera image data 344. After the subject support 102 was moved to the intermediate position 128, several other images were acquired. The memory 320 is further shown as containing the additional first camera image data 348 and the additional second camera image data 350. The memory 320 is further shown as containing the first camera three-dimensional image 352 that was calculated from the initial first camera image data 342 and the additional first camera image data 348.

The memory 320 is further shown as containing the second camera three-dimensional image 354 that was calculated from the initial second camera image data 344 and the additional second camera image data 350. The memory 320 is shown as optionally containing the additional three-dimensional image that was calculated from the first camera three-dimensional image 352 and the second camera three-dimensional image 354. The memory 320 is further shown as containing a composite three-dimensional image 358 that was calculated from the initial three-dimensional image 346, the first camera three-dimensional image 352, the second camera three-dimensional image 354 and optionally the additional three-dimensional image 356. The composite three-dimensional image 358 could be constructed in a patchwork manner taking various parts from the various three-dimensional images 346, 352, 354, 356. In some cases where the images overlap they are average. The memory 320 is further shown as containing an optional control signal 360 that was generated using the composite three-dimensional image 358. The composite three-dimensional image 358 could for example be useful for things such as positioning the subject as well as determining such things as the mass of the subject to determine if there will be an SAR problem in magnetic resonance imaging, or even detecting a probability of a collision of the subject 210 with a medical imaging system.

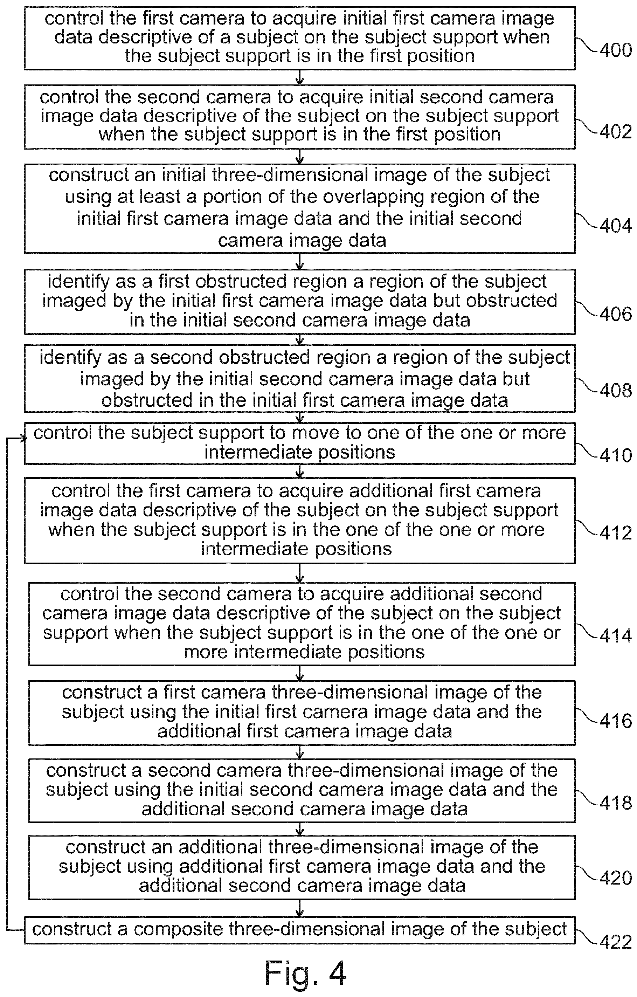

Fig. 4 shows a flowchart which illustrates a method of operating the medical system 300 of Fig. 3. The method is also applicable to the medical system 200 in Fig. 2. First, in step 400, the first camera 214 is controlled to acquire the initial first camera image data 342 when the subject support 206 is in the first position 324. Next, in step 402, the second camera 216 is controlled to acquire the initial second camera image data 344 when the subject support 206 is in the first position 324. Next, in step 406, the initial three-dimensional image 346 is constructed using an overlapping region of the initial first camera image data 342 and the initial second camera image data 344.

Next, in step 406, the first obstructed region 222 is identified in the initial second camera image data 344. Next, in step 208, the second obstructed region 224 is identified in the initial first camera image data 342. The following steps, 410, 412, 414, 416, 418, 420, 422 are repeated for at least one intermediate position. In step 410 the subject support is controlled to move to one of the one or more intermediate positions 328. Then, in step 412, the first camera 414 is controlled to acquire the additional first camera image data 348. Next, in step 414, the second camera 216 is controlled to acquire the additional second camera image data 350. Next, in step 416, the first camera three-dimensional image 352 is constructed from the initial first camera image data 342 and the additional first camera image data 348. Next, in step 418, the second camera three-dimensional image 354 is constructed from the initial second camera image data 344 and the additional second camera image data 350. Step 420 is an optional step. In step 420 the additional three-dimensional image 356 is constructed from the additional first camera image data 348 and the additional second camera image data 350. Finally, in step 422, the composite three-dimensional image 358 of the subject is constructed using the initial three-dimensional image 346, the first camera three-dimensional image 352, and the second camera three-dimensional image 354. In some examples the composite three-dimensional image 358 may also be constructed using the additional three-dimensional image 356. The first obstructed region 222 is at least partially replaced by the first camera three-dimensional image 352. The second obstructed region 224 is at least partially replaced by the second camera three-dimensional image 354. Steps, 410, 412, 414, 416, 418, 420, 422 may repeated to reduce the size of the obstructed regions 222, 224 and/or to reduce the noise in the composite three-dimensional image 358.

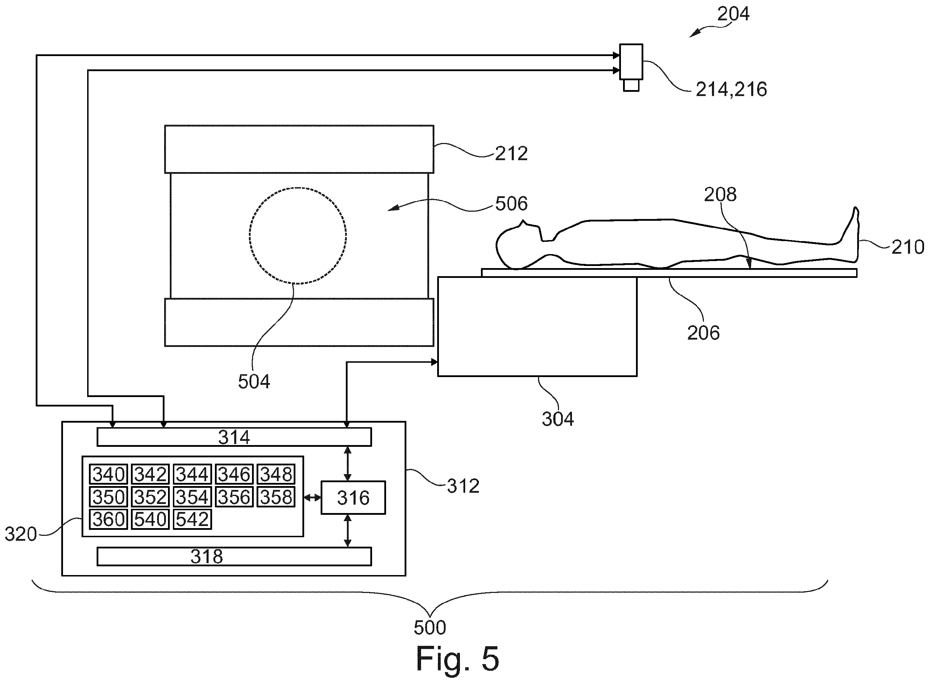

Fig. 5 illustrates a further example of a medical apparatus 500. The medical apparatus in Fig. 500 is similar to the medical apparatus 200 in Fig. 2 except there is now additionally a medical imaging system 212. The medical imaging system has a medical imaging volume 504 from which measurement can be acquired. The subject support 206 is configured for moving at least a portion of the subject 210 into the medical imaging volume 504. **In** this example the medical imaging system 212 is cylindrical and has a bore 506 which the subject 210 can be moved into using the subject support 206. This is however not necessary, not all medical imaging systems 212 need to be cylindrically symmetric as is illustrated in this Fig. Additionally, it should be noted that the camera 110 is now mounted onto the medical imaging system 302 and aimed at an oblique angle to the support surface 108. The memory 320 is further shown as containing measurement data 540 that has been acquired when the subject 210 was at least partially within the imaging volume 504. The memory 320 is also further shown as containing a medical image 542 that has been reconstructed from the measurement data 540.

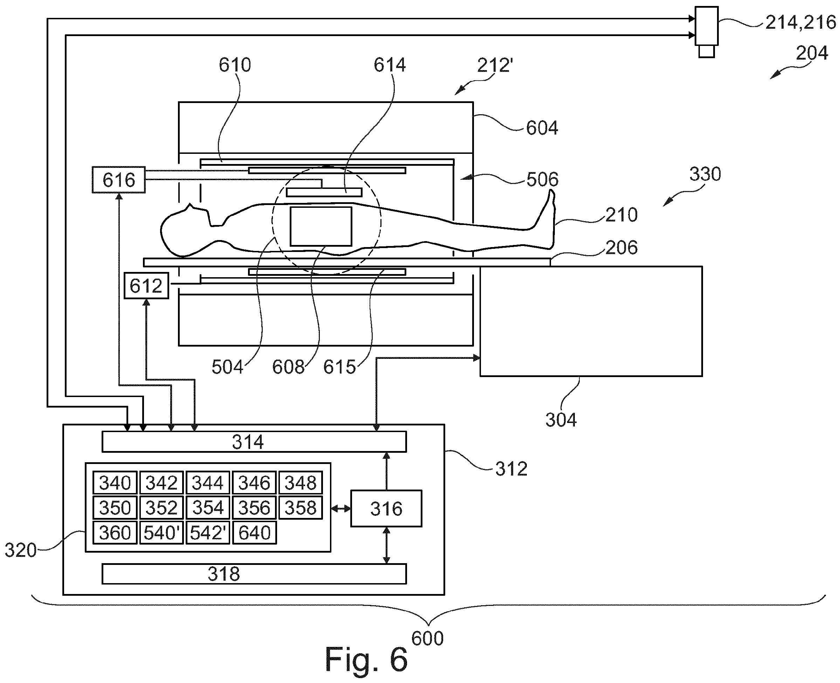

Fig. 6 shows a further example of a medical apparatus 600. The medical apparatus 600 in Fig. 6 is similar to the medical apparatus 500 in Fig. 5. However, in this example, the medical imaging system 212 is a magnetic resonance imaging system 212'. The magnetic resonance imaging system 212' comprises a main magnet 604, which may be referred to as the magnet. The magnet 404 is a superconducting cylindrical type magnet 604 with a bore 506 through it. The use of different types of magnets is also possible. Inside the cryostat of the cylindrical magnet, there is a collection of superconducting coils. Within the bore 506 of the cylindrical magnet 604 there is an imaging volume 504 where the magnetic field is strong and uniform enough to perform magnetic resonance imaging.

Within the bore 506 of the magnet there is also a set of magnetic field gradient coils 610 which is used for acquisition of magnetic resonance data to spatially encode magnetic spins within the imaging volume 504 of the magnet 604. The magnetic field gradient coils 610 are connected to a magnetic field gradient coil power supply 612. The magnetic field gradient coils 610 are intended to be representative. Typically, magnetic field gradient coils 610 contain three separate sets of coils for spatially encoding in three orthogonal spatial directions. A magnetic field gradient power supply supplies current to the magnetic field gradient coils. The current supplied to the magnetic field gradient coils 610 is controlled as a function of time and may be ramped or pulsed.

Adjacent to the imaging volume 504 in a magnetic resonance coil or antenna 614 that functions as radio-frequency antennas for manipulating the orientation of magnetic spins within the imaging volume 504 and for receiving radio transmissions from spins also within the imaging volume 504. The radio frequency coil may also be multiple coil elements. The radio frequency antenna may also be referred to as channel. The coil 614 is connected to a radio frequency transceiver 616. The coil 614 and radio frequency transceiver 616 may have separate transmitters and receivers. The coil 614 and the transceiver 616 form a radio-frequency system.

The if the coil 614 is made of multiple coil elements, they may be used to acquire magnetic resonance data separately. The coil elements may therefore be used for a parallel imaging magnetic resonance technique. An optional body coil 615 is also shown. The body coil 615 would be useful in the parallel imaging technique as it could take acquired data at the same time as the individual coil elements and be used for calculating a set of coil sensitivities. The magnetic resonance data may be acquired from within the imaging volume 504. The magnetic resonance data is an example of medical image data.

Within the bore 506 of the magnet 604 the subject support 206 is shown as supporting a portion of the subject 210 in the imaging volume 504. The subject support 206 is in the second position 330.

The transceiver 616, the actuator of the subject support 604, the two cameras 214, 216, actuator 304, and the gradient controller 612 are shown as being connected to the hardware interface 314 of the computer system 312. Within the memory 320 are located machine-executable instructions 140.

The computer memory 320 is further shown as containing pulse sequence commands 640. The pulse sequence commands 640 are either instructions or data which can be transformed into instructions which may be used to control the magnetic resonance imaging system 212' to acquire measurement data 540. In this case, the measurement data 540 is k-space data 540'. In this example the medical image 542 is a magnetic resonance image 542'.

Within the imaging volume 504 there is a field of view 608. The field of view 608 may for example have been identified using the composite three-dimensional image 358 before the subject was moved into the second position 330.

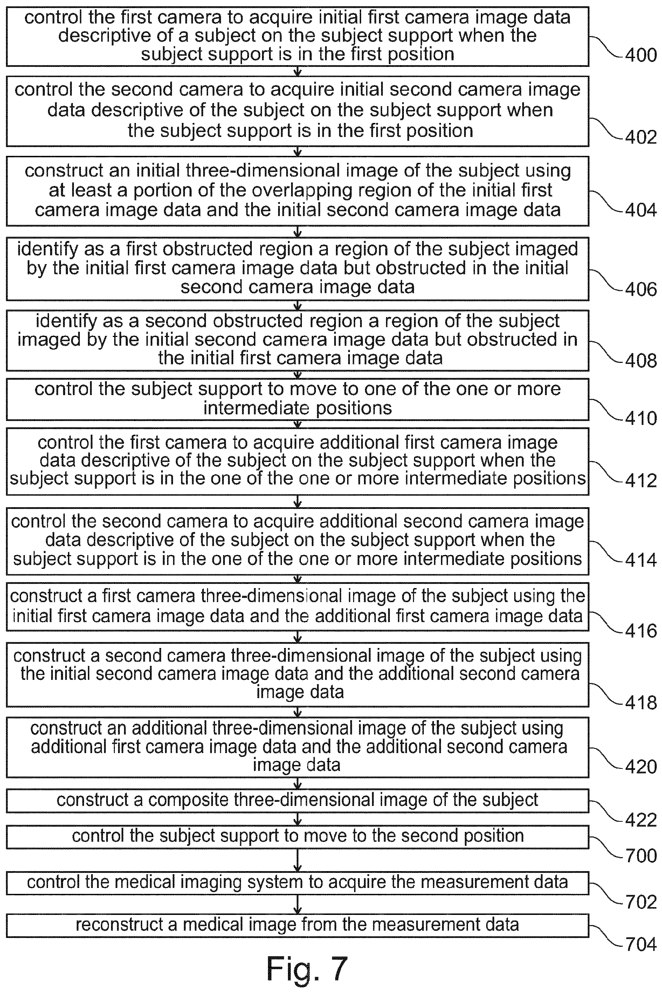

Fig. 7 shows a flowchart which illustrates a method that was similar to the method illustrated in Fig. 4. In the method illustrated in Fig. 7 method steps 400-422 are performed first. After step 422, step 600 is performed. In step 600 the subject support 206 is moved into the second position 330. Next, in step 602, the medical imaging system 212 is controlled to acquire the measurement data 642. In this example the measurement data 642 is k-space data. Finally, in step 604, the measurement data 642 is reconstructed into a medical image 644. In this example the medical image would be a magnetic resonance image 644.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: three-dimensional image
- 102: obstructed region of three-dimensional image
- 200: medical system
- 202: top view
- 204: side view
- 206: subject support
- 208: support surface
- 210: subject
- 212: medical imaging system
- 212': magnetic resonance imaging system
- 214: first camera
- 216: second camera
- 218: predetermined path
- 220: baseline
- 222: first obstructed region
- 224: second obstructed region
- 226: width of subject support
- 300: medical system
- 304: actuator
- 312: computer
- 314: hardware interface
- 316: computational system
- 318: user interface
- 320: memory
- 322: height above support surface
- 324: position of subject support in first position
- 326: position of subject support in second position
- 328: position of subject support in intermediate position
- 330: displacement between subject support in first and second positions
- 332: displacement between subject support in first and intermediate position
- 340: machine executable instructions
- 342: initial first camera image data
- 344: initial second camera image data
- 346: initial three-dimensional image
- 348: additional first camera image data
- 350: additional second camera image data
- 352: first camera three-dimensional image
- 354: second camera three-dimensional image
- 356: additional three-dimensional image
- 358: composite three-dimensional image
- 360: control signal
- 400: control the first camera to acquire initial first camera image data descriptive of a subject on the subject support when the subject support is in the first position
- 402: control the second camera to acquire initial second camera image data descriptive of the subject on the subject support when the subject support is in the first position
- 404: construct an initial three-dimensional image of the subject using at least a portion of the overlapping region of the initial first camera image data and the initial second camera image data
- 406: identify as a first obstructed region a region of the subject imaged by the initial first camera image data but obstructed in the initial second camera image data
- 408: identify as a second obstructed region a region of the subject imaged by the initial second camera image data but obstructed in the initial first camera image data
- 410: control the subject support to move to one of the one or more intermediate positions
- 412: control the first camera to acquire additional first camera image data descriptive of the subject on the subject support when the subject support is in the one of the one or more intermediate positions
- 414: control the second camera to acquire additional second camera image data descriptive of the subject on the subject support when the subject support is in the one of the one or more intermediate positions
- 416: construct a first camera three-dimensional image of the subject using the initial first camera image data and the additional first camera image data
- 418: construct a second camera three-dimensional image of the subject using the initial second camera image data and the additional second camera image data
- 420: construct an additional three-dimensional image of the subject using additional first camera image data and the additional second camera image data
- 422: construct a composite three-dimensional image of the subject
- 500: medical system
- 504: imaging volume
- 506: bore of medical imaging system
- 540: measurement data
- 540': k-space data
- 542: medical image
- 542': magnetic resonance image
- 600: medical system
- 604: main magnet
- 608: field of view
- 610: magnetic field gradient coils
- 612: gradient coil power supply
- 614: coil
- 615: body coil
- 616: transceiver
- 640: pulse sequence commands
- 700: control the subject support to move to the second position
- 702: control the medical imaging system to acquire the measurement data
- 704: preferably reconstruct a medical image from the measurement data

## Claims

1. A medical system (200, 300, 500. 600) comprising,
- a subject support (206), wherein the subject support comprises a support surface (208) for receiving a subject (210),
- a first camera (214) configured for imaging a first portion of the support surface;
- a second camera (216) configured for imaging a second portion of the support surface, wherein the first portion and the second portion comprise an overlapping region, wherein the subject support is movable relative to the first camera and the second camera along a predetermined path (218) between a first position (324), at least one intermediate position (326), and a second position (328), wherein each of the one or more intermediate positions is located between the first position and the second position;
- a memory (320) containing machine executable instructions (340); and
- a computational system (316) configured for controlling the medical system, wherein execution of the machine executable instructions causes the computational system to:
- control (400) the first camera to acquire initial first camera image data (342) descriptive of a subject on the subject support when the subject support is in the first position;
- control (402) the second camera to acquire initial second camera image data (344) descriptive of the subject on the subject support when the subject support is in the first position;
- construct (404) an initial three-dimensional image (346) of the subject using at least a portion of the overlapping region of the initial first camera image data and the initial second camera image data;
- identify (406) as a first obstructed region (222) a region of the subject imaged by the initial first camera image data but obstructed in the initial second camera image data; and
- identify (408) as a second obstructed region (224) a region of the subject imaged by the initial second camera image data but obstructed in the initial first camera image data;
wherein execution of the machine executable instructions further causes the computational system to perform the following at least once:
- control (410) the subject support to move to one of the one or more intermediate positions;
- control (412) the first camera to acquire additional first camera image data (348) descriptive of the subject on the subject support when the subject support is in the one of the one or more intermediate positions;
- control (414) the second camera to acquire additional second camera image data (350) descriptive of the subject on the subject support when the subject support is in the one of the one or more intermediate positions;
- construct (416) a first camera three-dimensional image (352) of the subject using the initial first camera image data and the additional first camera image data;
- construct (418) a second camera three-dimensional image (354) of the subject using the initial second camera image data and the additional second camera image data;
- construct (422) a composite three-dimensional image (358) of the subject using the initial three-dimensional image, the first camera three-dimensional image, and the second camera three-dimensional image, wherein the first obstructed region is at least partially replaced by the first camera three-dimensional image, and wherein the second obstructed region is at least partially replaced by the second camera three-dimensional image.

2. The medical system of claim 1, wherein execution of the machine executable instructions further causes the computational system to additionally perform the following at least once: construct (420) an additional three-dimensional image (356) of the subject using additional first camera image data and the additional second camera image data, and wherein the composite three-dimensional image is additional constructed using the additional three-dimensional image of the subject.

3. The medical system of claim 2, wherein the composite three-dimensional image comprises average regions constructed by averaging overlapping regions between two or more of the following: the initial three-dimensional image, the first camera three-dimensional image, the second camera three-dimensional image, the additional three-dimensional image, and previous iterations of the composite three-dimensional image.

4. The medical system of any one of the preceding claims, wherein the memory further comprises an anatomical key point module configured to output a set of anatomical key points for the subject in response to receiving any one of the following: the initial first camera image data, the initial second camera image data, the additional first camera image data, and the additional second camera image data, wherein execution of the machine executable instructions further causes the computational system to:
- receive initial first camera key point data in response to inputting the initial first camera image data into the anatomical key point module; and
- receive initial second camera key point data in response to inputting the initial second camera image data into the anatomical key point module.

5. The medical system of claim 4, wherein execution of the machine executable instructions further causes the computational system to calculate the one of the one or more intermediate positions using a location of anatomical key points of the initial first camera key point data and/or using a location of anatomical key points of the initial second camera key point data.

6. The medical system of claim 4 or 5, wherein execution of the machine executable instructions further causes the computational system to:
- receive image guided therapy position data descriptive of predefined anatomical key points of the subject relative to the subject support;
- detect a subject positioning error by comparing the predefined anatomical key points to the initial first camera key point data and/or the initial second camera key point data; and
- provide a warning signal if the subject positioning error is detected.

7. The medical system of claim 4, 5, or 6, wherein execution of the machine executable instructions further causes the computational system to:
- receive additional first camera key point data in response to inputting the additional first camera image data into the anatomical key point module;
- receive additional second camera key point data in response to inputting the additional second camera image data into the anatomical key point module;
- detect a subject motion condition between the first position of the subject support and the one of the one or more intermediate position by detecting a difference between the initial first camera key point data and the additional first camera key point data and/or a difference between the initial second camera key point data and the additional second camera key point data;
- exclude at least a portion of the first camera three-dimensional image and/or the second camera three-dimensional image from the composite three-dimensional image if the subject motion condition is detected.

8. The medical system any one of the preceding claims, wherein the first camera and the second camera form a stereo baseline (220) perpendicular to the predetermined path.

9. The medical system of any one claim 8, wherein the subject support has a maximum width (226) perpendicular to the predetermined path, wherein the stereo baseline is between 10% and 200% of the maximum width of the subject support, wherein the stereo baseline is preferably between 50% and 150% of the maximum width of the subject support.

10. The medical system of any one of the preceding claims, wherein execution of the machine executable instruction further causes the computational system to identify at least one stationary object region by detecting stationary voxels between the initial first camera image data and the additional first camera image data and/or by detecting stationary voxels between the initial second camera image data and the additional second camera image data, and wherein the at least one stationary object region is excluded from the composite three-dimensional image of the subject.

11. The medical system of any one of the preceding claims, wherein the medical system further comprises a medical imaging system (212, 212') for acquiring measurement data (540, 540') from an imaging volume (504), wherein at least a portion of the support surface is within the imaging volume when the subject support is within the second position, wherein execution of the machine executable instructions further causes the computational system to:
- control (700) the subject support to move to the second position;
- control (702) the medical imaging system to acquire the measurement data; and
- preferably (704) reconstruct a medical image (542, 542') from the measurement data.

12. The medical system of claim 10 or 11, wherein execution of the machine executable instructions further causes the computational system to perform any one of the following: calculate a isocenter for the subject, determine a subject position and/or orientation, perform collision risk prediction of the subject with the medical imaging system, calculate a SAR estimate for the subject, calculate a subject weight, calculate a subject height, and combinations thereof.

13. The medical system of claim 10, 11, or 12, wherein the medical imaging system is any one of the following: a magnetic resonance imaging system (212'), a position emission tomography system, a single photon emission tomography system, a computed tomography system, a combined positron emission tomography and magnetic resonance imaging system, a combined positron emission tomography and computed tomography system, a combined computer tomography and radiation therapy system, a combined computed tomography and positron emission tomography system, a combined magnetic resonance imaging system and radiation therapy system, and an image guided radiation therapy system.

14. A method of controlling a medical system (200, 300, 500, 600), wherein the medical system comprises: a subject support (206), wherein the subject support comprises a support surface (208) for receiving a subject (210); a first camera (214) configured for imaging a first portion of the support surface; and a second camera (216) configured for imaging a second portion of the support surface, wherein the first portion and the second portion comprise an overlapping region, wherein the subject support is movable relative to the first camera and the second camera along a predetermined path (218) between a first position (324), at least one intermediate position (326), and a second position (328), wherein each of the one or more intermediate positions is located between the first position and the second position;
wherein the method comprises:
- controlling (400) the first camera to acquire initial first camera image data (342) descriptive of a subject on the subject support when the subject support is in the first position;
- controlling (402) the second camera to acquire initial second camera image (344) data descriptive of the subject on the subject support when the subject support is in the first position;
- constructing (404) an initial three-dimensional image (346) of the subject using at least a portion of the overlapping region of the initial first camera image data and the initial second camera image data;
- identify (406) as a first obstructed region (222) a region of the subject imaged by the initial first camera image data but obstructed in the initial second camera image data; and
- identify (408) as a second obstructed region (224) a region of the subject imaged by the initial second camera image data but obstructed in the initial first camera image data;
wherein execution of the machine executable instructions further causes the computational system to perform the following at least once:
- controlling (410) the subject support to move to one of the one or more intermediate positions;
- controlling (412) the first camera to acquire additional first camera image data (348) descriptive of the subject on the subject support when the subject support is in the one of the one or more intermediate positions;
- controlling (414) the second camera to acquire additional second camera image data (350) descriptive of the subject on the subject support when the subject support is in the one of the one or more intermediate positions;
- constructing (416) a first camera three-dimensional image (352) of the subject using the initial first camera image data and the additional first camera image data;
- constructing (418) a second camera three-dimensional image (354) of the subject using the initial second camera image data and the additional second camera image data;
- constructing (422) a composite three-dimensional image (358) of the subject using the initial three-dimensional image, the first camera three-dimensional image, and the second camera three-dimensional image, wherein the first obstructed region is at least partially replaced by the first camera three-dimensional image, and wherein the second obstructed region is at least partially replaced by the second camera three-dimensional image.

15. A computer program comprising machine executable instructions (340) for execution by a computational system (316) controlling a medical system (200, 300, 500, 600), wherein the medical system comprises: a subject support ( 206), wherein the subject support comprises a support surface (208) for receiving a subject (210); a first camera (214) configured for imaging a first portion of the support surface; and a second camera (216) configured for imaging a second portion of the support surface, wherein the first portion and the second portion comprise an overlapping region, wherein the subject support is movable relative to the first camera and the second camera along a predetermined path (218) between a first position (324), at least one intermediate position (326), and a second position (328), wherein each of the one or more intermediate positions is located between the first position and the second position;
wherein execution of the machine executable instructions causes the computational system to:
- control (400) the first camera to acquire initial first camera image data (342) descriptive of a subject on the subject support when the subject support is in the first position;
- control (402) the second camera to acquire initial second camera image data (344) descriptive of the subject on the subject support when the subject support is in the first position;
- construct (404) an initial three-dimensional image (346) of the subject using at least a portion of the overlapping region of the initial first camera image data and the initial second camera image data;
- identify (406) as a first obstructed region (222) a region of the subject imaged by the initial first camera image data but obstructed in the initial second camera image data; and
- identify (408) as a second obstructed region (224) a region of the subject imaged by the initial second camera image data but obstructed in the initial first camera image data;
wherein execution of the machine executable instructions further causes the computational system to perform the following at least once:
- control (410) the subject support to move to one of the one or more intermediate positions;
- control (412) the first camera to acquire additional first camera image data (348) descriptive of the subject on the subject support when the subject support is in the one of the one or more intermediate positions;
- control (414) the second camera to acquire additional second camera image data (350) descriptive of the subject on the subject support when the subject support is in the one of the one or more intermediate positions;
- construct (416) a first camera three-dimensional image (352) of the subject using the initial first camera image data and the additional first camera image data;
- construct (418) a second camera three-dimensional image (354) of the subject using the initial second camera image data and the additional second camera image data;
- construct (422) a composite three-dimensional image (358) of the subject using the initial three-dimensional image, the first camera three-dimensional image, and the second camera three-dimensional image, wherein the first obstructed region is at least partially replaced by the first camera three-dimensional image, and wherein the second obstructed region is at least partially replaced by the second camera three-dimensional image.

## Patentansprüche

1. Medizinisches System (200, 300, 500, 600), umfassend:
- eine Patientenliege (206), wobei die Patientenliege eine Auflagefläche (208) zum Aufnehmen eines Subjekts (210) umfasst,
- eine erste Kamera (214), die für Bildgebung eines ersten Abschnitts der Auflagefläche konfiguriert ist;
- eine zweite Kamera (216), die für Bildgebung eines zweiten Abschnitts der Auflagefläche konfiguriert ist, wobei der erste und der zweite Abschnitt einen Überlappungsbereich umfassen, wobei die Patientenliege relativ zur ersten Kamera und zweiten Kamera entlang eines vorbestimmten Pfades (218) zwischen einer ersten Position (324), mindestens einer Zwischenposition (326) und einer zweiten Position (328) beweglich ist, wobei jede der einen oder mehreren Zwischenpositionen zwischen der ersten Position und der zweiten Position gelegen ist;
- einen Speicher (320), der maschinenausführbare Anweisungen (340) enthält, und
- ein Rechensystem (316), das so konfiguriert ist, dass es das medizinische Instrument steuert, wobei Ausführung der maschinenausführbaren Anweisungen das Rechensystem veranlasst zum:
- Steuern (400) der ersten Kamera zum Erfassen anfänglicher Bilddaten (342) der ersten Kamera, die für ein Subjekt auf der Patientenliege beschreibend sind, wenn sich die Patientenliege in der ersten Position befindet;
- Steuern (402) der zweiten Kamera zum Erfassen anfänglicher Bilddaten (344) der zweiten Kamera, die für das Subjekt auf der Patientenliege beschreibend sind, wenn sich die Patientenliege in der ersten Position befindet;
- Konstruieren (404) eines anfänglichen dreidimensionales Bildes (346) des Subjekts unter Verwendung mindestens eines Abschnitts des Überlappungsbereichs der anfänglichen Bilddaten der ersten Kamera und der anfänglichen Bilddaten der zweiten Kamera;
- Identifizieren (406) als einen ersten verdeckten Bereich (222) eines Bereichs des durch die anfänglichen Bilddaten der ersten Kamera abgebildeten Subjekts, der in den anfänglichen Bilddaten der zweiten Kamera verdeckt ist; und
- Identifizieren (408) als einen zweiten verdeckten Bereich (224) eines Bereichs des durch die anfänglichen Bilddaten der zweiten Kamera abgebildeten Subjekts, der in den anfänglichen Bilddaten der ersten Kamera verdeckt ist;
wobei Ausführung der maschinenausführbaren Anweisungen das Rechensystem weiter veranlasst, mindestens eines von Folgendem durchzuführen:
- Steuern (410) der Patientenliege, um sich zu einer der einen oder mehreren Zwischenpositionen zu bewegen;
- Steuern (412) der ersten Kamera zum Erfassen zusätzlicher Bilddaten (348) der ersten Kamera, die für das Subjekt auf der Patientenliege beschreibend sind, wenn sich die Patientenliege in einer der einen oder mehreren Zwischenpositionen befindet;
- Steuern (414) der zweiten Kamera zum Erfassen zusätzlicher Bilddaten (350) der zweiten Kamera, die für das Subjekt auf der Patientenliege beschreibend sind, wenn sich die Patientenliege in einer der einen oder mehreren Zwischenpositionen befindet;
- Konstruieren (416) eines dreidimensionalen Bildes (352) des Subjekts der ersten Kamera unter Verwendung der anfänglichen Bilddaten der ersten Kamera und der zusätzlichen Bilddaten der ersten Kamera;
- Konstruieren (418) eines dreidimensionalen Bildes (354) des Subjekts der zweiten Kamera unter Verwendung der anfänglichen Bilddaten der zweiten Kamera und der zusätzlichen Bilddaten der zweiten Kamera;
- Konstruieren (422) eines zusammengesetzten dreidimensionalen Bildes (358) des Subjekts unter Verwendung des anfänglichen dreidimensionalen Bildes, des dreidimensionalen Bildes der ersten Kamera und des dreidimensionalen Bildes der zweiten Kamera, wobei der erste verdeckte Bereich mindestens teilweise durch das dreidimensionale Bild der ersten Kamera und der zweite verdeckte Bereich mindestens teilweise durch das dreidimensionale Bild der zweiten Kamera ersetzt wird.

2. Medizinisches System nach Anspruch 1, wobei Ausführung der maschinenausführbaren Anweisungen das Rechensystem weiter veranlasst, Folgendes mindestens einmal durchzuführen: Konstruieren (420) eines zusätzlichen dreidimensionalen Bildes (356) des Subjekts unter Verwendung zusätzlicher Bilddaten der ersten Kamera und zusätzlicher Bilddaten der zweiten Kamera, wobei das zusammengesetzte dreidimensionale Bild zusätzlich unter Verwendung des zusätzlichen dreidimensionalen Bildes des Subjekts konstruiert wird.

3. Medizinisches System nach Anspruch 2, wobei das zusammengesetzte dreidimensionale Bild Mittelwertbereiche umfasst, die durch Mitteln überlappender Bereiche zwischen zwei oder mehr des Folgenden konstruiert werden: dem anfänglichen dreidimensionalen Bild, dem dreidimensionalen Bild der ersten Kamera, dem dreidimensionalen Bild der zweiten Kamera, dem zusätzlichen dreidimensionalen Bild und früheren Iterationen des zusammengesetzten dreidimensionalen Bildes.

4. Medizinisches System nach einem der vorstehenden Ansprüche, wobei der Speicher weiter ein Modul für anatomische Schlüsselpunkte umfasst, das so konfiguriert ist, dass es einen Satz anatomischer Schlüsselpunkte für das Subjekt ausgibt, als Reaktion auf Empfangen eines von Folgendem: der anfänglichen Bilddaten der ersten Kamera, der anfänglichen Bilddaten der zweiten Kamera, der zusätzlichen Bilddaten der ersten Kamera und der zusätzlichen Bilddaten der zweiten Kamera, wobei Ausführung der maschinenausführbaren Anweisungen das Rechensystem weiter zu Folgendem veranlasst:
- Empfangen von anfänglichen Schlüsselpunktdaten der ersten Kamera als Reaktion auf Eingabe der anfänglichen Bilddaten der ersten Kamera in das Modul für anatomische Schlüsselpunkte; und
- Empfangen von anfänglichen Schlüsselpunktdaten der zweiten Kamera als Reaktion auf Eingabe der anfänglichen Bilddaten der zweiten Kamera in das Modul für anatomische Schlüsselpunkte.

5. Medizinisches System nach Anspruch 4, wobei Ausführung der maschinenausführbaren Anweisungen das Rechensystem weiter veranlasst, die eine oder mehreren Zwischenpositionen unter Verwendung einer Lage von anatomischen Schlüsselpunkten der anfänglichen Schlüsselpunktdaten der ersten Kamera und/oder unter Verwendung einer Lage anatomischer Schlüsselpunkte der anfänglichen Schlüsselpunktdaten der zweiten Kamera zu berechnen.

6. Medizinisches System nach Anspruch 4 oder 5, wobei Ausführung der maschinenausführbaren Anweisungen das Rechensystem weiter veranlasst zum:
- Empfangen von bildgeführten Therapiepositionsdaten, die für vordefinierte anatomische Schlüsselpunkte des Subjekts relativ zur Patientenauflage beschreibend sind;
- Erkennen eines Positionierungsfehlers des Subjekts durch Vergleichen der vordefinierten anatomischen Schlüsselpunkte mit den anfänglichen Schlüsselpunktdaten der ersten Kamera und/oder anfänglichen Schlüsselpunktdaten der zweiten Kamera; und
- Bereitstellen eines Warnsignals, wenn ein Positionierungsfehler des Subjekts erkannt wird.

7. Medizinisches System nach Anspruch 4, 5 oder 6, wobei Ausführung der maschinenausführbaren Anweisungen das Rechensystem weiter veranlasst zum:
- Empfangen zusätzlicher Schlüsselpunktdaten der ersten Kamera als Reaktion auf Eingabe der zusätzlichen Bilddaten der ersten Kamera in das Modul für anatomische Schlüsselpunkte;
- Empfangen zusätzlicher Schlüsselpunktdaten der zweiten Kamera als Reaktion auf Eingabe der zusätzlichen Bilddaten der zweiten Kamera in das Modul für anatomische Schlüsselpunkte;
- Erkennen eines Bewegungszustands des Subjekts zwischen der ersten Position der Patientenliege und der einen oder den mehreren Zwischenpositionen durch Erkennen einer Differenz zwischen den anfänglichen Schlüsselpunktdaten der ersten Kamera und den zusätzlichen Schlüsselpunktdaten der ersten Kamera und/oder einer Differenz zwischen den anfänglichen Schlüsselpunktdaten der zweiten Kamera und den zusätzlichen Schlüsselpunktdaten der zweiten Kamera;
- Ausschließen mindestens eines Abschnitts des dreidimensionalen Bildes der ersten Kamera und/oder des dreidimensionalen Bildes der zweiten Kamera aus dem zusammengesetzten dreidimensionalen Bild, wenn ein Bewegungszustand des Subjekts erkannt wird.

8. Medizinisches System nach einem der vorstehenden Ansprüche, wobei die erste Kamera und die zweite Kamera eine Stereobasislinie (220) senkrecht zum vorbestimmten Pfad bilden.

9. Medizinisches System nach Anspruch 8, wobei die Patientenliege eine maximale Breite (226) senkrecht zum vorbestimmten Pfad aufweist, wobei die Stereobasislinie zwischen 10 % und 200 % der maximalen Breite der Patientenliege beträgt, wobei die Stereobasislinie vorzugsweise zwischen 50 % und 150 % der maximalen Breite der Patientenliege beträgt.

10. Medizinisches System nach einem der vorstehenden Ansprüche, wobei Ausführung der maschinenausführbaren Anweisung das Rechensystem weiter veranlasst zum Identifizieren mindestens eines stationären Objektbereichs durch Erkennen stationärer Voxel zwischen den anfänglichen Bilddaten der ersten Kamera und den zusätzlichen Bilddaten der ersten Kamera und/oder durch Erkennen stationärer Voxel zwischen den anfänglichen Bilddaten der zweiten Kamera und den zusätzlichen Bilddaten der zweiten Kamera, und wobei der mindestens eine stationäre Objektbereich aus dem zusammengesetzten dreidimensionalen Bild des Subjekts ausgeschlossen wird.

11. Medizinisches System nach einem der vorstehenden Ansprüche, wobei das medizinische System weiter ein medizinisches Bildgebungssystem (212, 212') zum Erfassen von Messdaten (540, 540') aus einem Bildgebungsvolumen (504) umfasst, wobei sich mindestens ein Abschnitt der Auflagefläche innerhalb des Bildgebungsvolumens befindet, wenn sich die Patientenliege des Patienten in der zweiten Position befindet, und wobei Ausführung der maschinenausführbaren Anweisungen das Rechensystem weiter veranlasst zum:
- Steuern (700) der Patientenliege, um sich zu der zweiten Position zu bewegen;
- Steuern (702) des medizinischen Bildgebungssystems, um die Messdaten zu erfassen; und
- vorzugsweise Rekonstruieren (704) eines medizinischen Bildes (542, 542') aus den Messdaten.

12. Medizinisches System nach Anspruch 10 oder 11, wobei Ausführung der maschinenausführbaren Anweisungen das Rechensystem weiter veranlasst, eines von Folgendem durchzuführen: Berechnen eines Isozentrums für das Subjekt, Bestimmen einer Subjektposition und/oder -ausrichtung, Durchführen einer Kollisionsrisikoprognose des Subjekts mit dem medizinischen Bildgebungssystem, Berechnen einer SAR-Schätzung für das Subjekt, Berechnen des Gewichts des Subjekts, Berechnen der Größe des Subjekts und Kombinationen davon.

13. Medizinisches System nach Anspruch 10, 11 oder 12, wobei das medizinische Bildgebungssystem eines von Folgendem ist: ein Magnetresonanz-Bildgebungssystem (212'), ein Positronenemissionstomographiesystem, ein Einzelphotonenemissionstomographiesystem, ein Computertomographiesystem, ein kombiniertes Positronenemissionstomographie- und Magnetresonanz-Bildgebungssystem, ein kombiniertes Positronenemissionstomographie- und Computertomographiesystem, ein kombiniertes Computertomographie- und Strahlentherapiesystem, ein kombiniertes Computertomographie- und Positronenemissionstomographiesystem, ein kombiniertes Magnetresonanztomographie- und Strahlentherapiesystem oder ein bildgeführtes Strahlentherapiesystem.

14. Verfahren zum Steuern eines medizinischen Systems (200, 300, 500, 600), wobei das medizinische System Folgendes umfasst: eine Patientenliege (206), wobei die Patientenliege eine Auflagefläche (208) zum Aufnehmen eines Subjekts (210) umfasst; eine erste Kamera (214), die zur Bildgebung eines ersten Abschnitts der Auflagefläche konfiguriert ist; und eine zweite Kamera (216), die zur Bildgebung eines zweiten Abschnitts der Auflagefläche konfiguriert ist, wobei der erste Abschnitt und der zweite Abschnitt einen Überlappungsbereich umfassen, wobei die Patientenliege relativ zur ersten und zweiten Kamera entlang eines vorbestimmten Pfades (218) zwischen einer ersten Position (324), mindestens einer Zwischenposition (326) und einer zweiten Position (328) beweglich ist, wobei jede der einen oder mehreren Zwischenpositionen zwischen der ersten Position und der zweiten Position gelegen ist.
wobei das Verfahren Folgendes umfasst:
- Steuern (400) der ersten Kamera zum Erfassen anfänglicher Bilddaten (342) der ersten Kamera, die für ein Subjekt auf der Patientenliege beschreibend sind, wenn sich die Patientenliege in der ersten Position befindet;
- Steuern (402) der zweiten Kamera zum Erfassen anfänglicher Bilddaten (344) der zweiten Kamera, die für das Subjekt auf der Patientenliege beschreibend sind, wenn sich die Patientenliege in der ersten Position befindet;
- Konstruieren (404) eines anfänglichen dreidimensionales Bildes (346) des Subjekts unter Verwendung mindestens eines Abschnitts des Überlappungsbereichs der anfänglichen Bilddaten der ersten Kamera und der anfänglichen Bilddaten der zweiten Kamera;
- Identifizieren (406) als einen ersten verdeckten Bereich (222) eines Bereichs des durch die anfänglichen Bilddaten der ersten Kamera abgebildeten Subjekts, der in den anfänglichen Bilddaten der zweiten Kamera verdeckt ist; und
- Identifizieren (408) als einen zweiten verdeckten Bereich (224) eines Bereichs des durch die anfänglichen Bilddaten der zweiten Kamera abgebildeten Subjekts, der in den anfänglichen Bilddaten der ersten Kamera verdeckt ist;
wobei Ausführung der maschinenausführbaren Anweisungen das Rechensystem weiter veranlasst, mindestens eines von Folgendem durchzuführen:
- Steuern (410) der Patientenliege, um sich zu einer der einen oder mehreren Zwischenpositionen zu bewegen;
- Steuern (412) der ersten Kamera zum Erfassen zusätzlicher Bilddaten (348) der ersten Kamera, die für das Subjekt auf der Patientenliege beschreibend sind, wenn sich die Patientenliege in einer der einen oder mehreren Zwischenpositionen befindet;
- Steuern (414) der zweiten Kamera zum Erfassen zusätzlicher Bilddaten (350) der zweiten Kamera, die für das Subjekt auf der Patientenliege beschreibend sind, wenn sich die Patientenliege in einer der einen oder mehreren Zwischenpositionen befindet;
- Konstruieren (416) eines dreidimensionalen Bildes (352) des Subjekts der ersten Kamera unter Verwendung der anfänglichen Bilddaten der ersten Kamera und der zusätzlichen Bilddaten der ersten Kamera;
- Konstruieren (418) eines dreidimensionalen Bildes (354) des Subjekts der zweiten Kamera unter Verwendung der anfänglichen Bilddaten der zweiten Kamera und der zusätzlichen Bilddaten der zweiten Kamera;
- Konstruieren (422) eines zusammengesetzten dreidimensionalen Bildes (358) des Subjekts unter Verwendung des anfänglichen dreidimensionalen Bildes, des dreidimensionalen Bildes der ersten Kamera und des dreidimensionalen Bildes der zweiten Kamera, wobei der erste verdeckte Bereich mindestens teilweise durch das dreidimensionale Bild der ersten Kamera und der zweite verdeckte Bereich mindestens teilweise durch das dreidimensionale Bild der zweiten Kamera ersetzt wird.

15. Computerprogramm, umfassend maschinenausführbaren Anweisungen (340) zur Ausführung durch ein Rechensystem (316), das ein medizinisches System (200, 300, 500, 600) steuert, wobei das medizinische System Folgendes umfasst: eine Patientenliege (206), wobei die Patientenliege eine Auflagefläche (208) zum Aufnehmen eines Subjekts (210) umfasst; eine erste Kamera (214), die zur Bildgebung eines ersten Abschnitts der Auflagefläche konfiguriert ist; und eine zweite Kamera (216), die zur Bildgebung eines zweiten Abschnitts der Auflagefläche konfiguriert ist, wobei der erste Abschnitt und der zweite Abschnitt einen Überlappungsbereich umfassen, wobei die Patientenliege relativ zur ersten und zweiten Kamera entlang eines vorbestimmten Pfades (218) zwischen einer ersten Position (324), mindestens einer Zwischenposition (326) und einer zweiten Position (328) beweglich ist, wobei jede der einen oder mehreren Zwischenpositionen zwischen der ersten Position und der zweiten Position gelegen ist;
wobei Ausführung der maschinenausführbaren Anweisungen das Rechensystem veranlasst zum:
- Steuern (400) der ersten Kamera zum Erfassen anfänglicher Bilddaten (342) der ersten Kamera, die für ein Subjekt auf der Patientenliege beschreibend sind, wenn sich die Patientenliege in der ersten Position befindet;
- Steuern (402) der zweiten Kamera zum Erfassen anfänglicher Bilddaten (344) der zweiten Kamera, die für das Subjekt auf der Patientenliege beschreibend sind, wenn sich die Patientenliege in der ersten Position befindet;
- Konstruieren (404) eines anfänglichen dreidimensionales Bildes (346) des Subjekts unter Verwendung mindestens eines Abschnitts des Überlappungsbereichs der anfänglichen Bilddaten der ersten Kamera und der anfänglichen Bilddaten der zweiten Kamera;
- Identifizieren (406) als einen ersten verdeckten Bereich (222) eines Bereichs des durch die anfänglichen Bilddaten der ersten Kamera abgebildeten Subjekts, der in den anfänglichen Bilddaten der zweiten Kamera verdeckt ist; und
- Identifizieren (408) als einen zweiten verdeckten Bereich (224) eines Bereichs des durch die anfänglichen Bilddaten der zweiten Kamera abgebildeten Subjekts, der in den anfänglichen Bilddaten der ersten Kamera verdeckt ist;
wobei Ausführung der maschinenausführbaren Anweisungen das Rechensystem weiter veranlasst, mindestens eines von Folgendem durchzuführen:
- Steuern (410) der Patientenliege, um sich zu einer der einen oder mehreren Zwischenpositionen zu bewegen;
- Steuern (412) der ersten Kamera zum Erfassen zusätzlicher Bilddaten (348) der ersten Kamera, die für das Subjekt auf der Patientenliege beschreibend sind, wenn sich die Patientenliege in einer der einen oder mehreren Zwischenpositionen befindet;
- Steuern (414) der zweiten Kamera zum Erfassen zusätzlicher Bilddaten (350) der zweiten Kamera, die für das Subjekt auf der Patientenliege beschreibend sind, wenn sich die Patientenliege in einer der einen oder mehreren Zwischenpositionen befindet;
- Konstruieren (416) eines dreidimensionalen Bildes (352) des Subjekts der ersten Kamera unter Verwendung der anfänglichen Bilddaten der ersten Kamera und der zusätzlichen Bilddaten der ersten Kamera;
- Konstruieren (418) eines dreidimensionalen Bildes (354) des Subjekts der zweiten Kamera unter Verwendung der anfänglichen Bilddaten der zweiten Kamera und der zusätzlichen Bilddaten der zweiten Kamera;
- Konstruieren (422) eines zusammengesetzten dreidimensionalen Bildes (358) des Subjekts unter Verwendung des anfänglichen dreidimensionalen Bildes, des dreidimensionalen Bildes der ersten Kamera und des dreidimensionalen Bildes der zweiten Kamera, wobei der erste verdeckte Bereich mindestens teilweise durch das dreidimensionale Bild der ersten Kamera und der zweite verdeckte Bereich mindestens teilweise durch das dreidimensionale Bild der zweiten Kamera ersetzt wird.

## Revendications

1. Système médical (200, 300, 500, 600) comprenant :
- un support de sujet (206), dans lequel le support de sujet comprend une surface de support (208) destinée à recevoir un sujet (210),
- une première caméra (214) configurée pour imager une première partie de la surface de support ;
- une seconde caméra (216) configurée pour imager une seconde partie de la surface de support, dans lequel la première partie et la seconde partie comprennent une région de chevauchement, dans lequel le support de sujet est mobile par rapport à la première caméra et à la seconde caméra le long d'un chemin prédéterminé (218) entre une première position (324), au moins une position intermédiaire (326) et une seconde position (328), dans lequel chacune des une ou plusieurs positions intermédiaires est située entre la première position et la seconde position ;
- une mémoire (320) contenant des instructions exécutables par machine (340) ; et
- un système de calcul (316) configuré pour commander le système médical, dans lequel l'exécution des instructions exécutables par machine amène le système de calcul à :
- commander (400) la première caméra pour acquérir des données d'image de première caméra initiales (342) descriptives d'un sujet sur le support de sujet lorsque le support de sujet est dans la première position ;
- commander (402) la seconde caméra pour acquérir des données d'image de seconde caméra initiales (344) descriptives du sujet sur le support de sujet lorsque le support de sujet est dans la première position ;
- construire (404) une image tridimensionnelle initiale (346) du sujet en utilisant au moins une partie de la région de chevauchement des données d'image de première caméra initiales et des données d'image de seconde caméra initiales ;
- l'identification (406) en tant que première région obstruée (222) d'une région du sujet imagée par les données d'image de première caméra initiales mais obstruée dans les données d'image de seconde caméra initiales ; et
- l'identification (408) en tant que seconde région obstruée (224) d'une région du sujet imagée par les données d'image de seconde caméra initiales mais obstruée dans les données d'image de première caméra initiales ;
dans lequel l'exécution des instructions exécutables par machine amène en outre le système de calcul à réaliser ce qui suit au moins une fois :
- commander (410) le support de sujet pour qu'il se déplace vers l'une des une ou plusieurs positions intermédiaires ;
- commander (412) la première caméra pour acquérir des données d'image de première caméra supplémentaires (348) descriptives du sujet sur le support de sujet lorsque le support de sujet est dans ladite position des une ou plusieurs positions intermédiaires ;
- commander (414) la seconde caméra pour acquérir des données d'image de seconde caméra supplémentaires (350) descriptives du sujet sur le support de sujet lorsque le support de sujet est dans ladite position des une ou plusieurs positions intermédiaires ;
- construire (416) une image tridimensionnelle de première caméra (352) du sujet en utilisant les données d'image de première caméra initiales et les données d'image de première caméra supplémentaires ;
- construire (418) une image tridimensionnelle de seconde caméra (354) du sujet en utilisant les données d'image de seconde caméra initiales et les données d'image de seconde caméra supplémentaires ;
- construire (422) une image tridimensionnelle composite (358) du sujet en utilisant l'image tridimensionnelle initiale, l'image tridimensionnelle de première caméra et l'image tridimensionnelle de seconde caméra, dans lequel la première région obstruée est au moins partiellement remplacée par l'image tridimensionnelle de première caméra, et dans lequel la seconde région obstruée est au moins partiellement remplacée par l'image tridimensionnelle de seconde caméra.

2. Système médical selon la revendication 1, dans lequel l'exécution des instructions exécutables par machine amène en outre le système de calcul à réaliser en plus ce qui suit au moins une fois : construire (420) une image tridimensionnelle supplémentaire (356) du sujet en utilisant des données d'image de première caméra supplémentaires et les données d'image de seconde caméra supplémentaires, et dans lequel l'image tridimensionnelle composite est construite en plus en utilisant l'image tridimensionnelle supplémentaire du sujet.

3. Système médical selon la revendication 2, dans lequel l'image tridimensionnelle composite comprend des régions moyennes construites en faisant la moyenne de régions de chevauchement entre deux de ce qui suit ou plus : l'image tridimensionnelle initiale, l'image tridimensionnelle de première caméra, l'image tridimensionnelle de seconde caméra, l'image tridimensionnelle supplémentaire et des itérations précédentes de l'image tridimensionnelle composite.

4. Système médical selon l'une quelconque des revendications précédentes, dans lequel la mémoire comprend en outre un module de points clés anatomiques configuré pour émettre un ensemble de points clés anatomiques pour le sujet en réponse à la réception de l'un quelconque de ce qui suit : les données d'image de première caméra initiales, les données d'image de seconde caméra initiales, les données d'image de première caméra supplémentaires et les données d'image de seconde caméra supplémentaires, dans lequel l'exécution des instructions exécutables par machine amène en outre le système de calcul à :
- recevoir des données de points clés de première caméra initiales en réponse à l'entrée des données d'image de première caméra initiales dans le module de points clés anatomiques ; et
- recevoir des données de points clés de seconde caméra initiales en réponse à l'entrée des données d'image de seconde caméra initiales dans le module de points clés anatomiques.

5. Système médical selon la revendication 4, dans lequel l'exécution des instructions exécutables par machine amène en outre le système de calcul à calculer ladite position des une ou plusieurs positions intermédiaires en utilisant un emplacement de points clés anatomiques des données de points clés de première caméra initiales et/ou en utilisant un emplacement de points clés anatomiques des données de points clés de seconde caméra initiales.

6. Système médical selon la revendication 4 ou 5, dans lequel l'exécution des instructions exécutables par machine amène en outre le système de calcul à :
- recevoir des données de position de thérapie guidée par l'image descriptives de points clés anatomiques prédéfinis du sujet par rapport au support de sujet ;
- détecter une erreur de positionnement de sujet en comparant les points clés anatomiques prédéfinis aux données de points clés de première caméra initiales et/ou aux données de points clés de seconde caméra initiales ; et
- fournir un signal d'avertissement si l'erreur de positionnement de sujet est détectée.

7. Système médical selon la revendication 4, 5 ou 6, dans lequel l'exécution des instructions exécutables par machine amène en outre le système de calcul à :
- recevoir des données de points clés de première caméra supplémentaires en réponse à l'entrée des données d'image de première caméra supplémentaires dans le module de points clés anatomiques ;
- recevoir des données de points clés de seconde caméra supplémentaires en réponse à l'entrée des données d'image de seconde caméra supplémentaires dans le module de points clés anatomiques ;
- détecter un état de mouvement de sujet entre la première position du support de sujet et ladite position des une ou plusieurs positions intermédiaires en détectant une différence entre les données de points clés de première caméra initiales et les données de points clés de première caméra supplémentaires et/ou une différence entre les données de points clés de seconde caméra supplémentaires et les données de points clés de seconde caméra supplémentaires ;
- exclure au moins une partie de l'image tridimensionnelle de première caméra et/ou de l'image tridimensionnelle de seconde caméra à partir de l'image tridimensionnelle composite si l'état de mouvement de sujet est détecté.

8. Système médical selon l'une quelconque des revendications précédentes, dans lequel la première caméra et la seconde caméra forment une ligne de base stéréo (220) perpendiculaire au chemin prédéterminé.

9. Système médical selon la revendication 8, dans lequel le support de sujet présente une largeur maximale (226) perpendiculaire au chemin prédéterminé, dans lequel la ligne de base stéréo est comprise entre 10 % et 200 % de la largeur maximale du support de sujet, dans lequel la ligne de base stéréo est de préférence comprise entre 50 % et 150 % de la largeur maximale du support de sujet.

10. Système médical selon l'une quelconque des revendications précédentes, dans lequel l'exécution de l'instruction exécutable par machine amène en outre le système de calcul à identifier au moins une région d'objet fixe en détectant des voxels fixes entre les données d'image de première caméra initiales et les données d'image de première caméra supplémentaires et/ou en détectant des voxels fixes entre les données d'image de seconde caméra initiales et les données d'image de seconde caméra supplémentaires, et dans lequel la au moins une région d'objet fixe est exclue de l'image tridimensionnelle composite du sujet.

11. Système médical selon l'une quelconque des revendications précédentes, dans lequel le système médical comprend en outre un système d'imagerie médicale (212, 212') pour acquérir des données de mesure (540, 540') à partir d'un volume d'imagerie (504), dans lequel au moins une partie de la surface de support est dans le volume d'imagerie lorsque le support de sujet est dans la seconde position, dans lequel l'exécution des instructions exécutables par machine amène en outre le système de calcul à :
- commander (700) le support de sujet pour qu'il se déplace vers la seconde position ;
- commander (702) le système d'imagerie médicale pour acquérir les données de mesure ; et
- de préférence (704) reconstruire une image médicale (542, 542') à partir des données de mesure.

12. Système médical selon la revendication 10 ou 11, dans lequel l'exécution des instructions exécutables par machine amène en outre le système de calcul à réaliser l'un quelconque de ce qui suit : calculer un isocentre pour le sujet, déterminer une position et/ou une orientation de sujet, réaliser une prédiction de risque de collision du sujet avec le système d'imagerie médicale, calculer une estimation de SAR pour le sujet, calculer un poids de sujet, calculer une taille de sujet, et des combinaisons de ceux-ci.

13. Système médical selon la revendication 10, 11 ou 12, dans lequel le système d'imagerie médicale est l'un quelconque de ce qui suit : un système d'imagerie par résonance magnétique (212'), un système de tomographie par émission de positons, un système de tomographie par émission de photons uniques, un système de tomodensitométrie, un système combiné de tomographie par émission de positons et d'imagerie par résonance magnétique, un système combiné de tomographie par émission de positons et de tomodensitométrie, un système combiné de tomodensitométrie et de radiothérapie, un système combiné de tomodensitométrie et de tomographie par émission de positons, un système combiné d'imagerie par résonance magnétique et de radiothérapie, et un système de radiothérapie guidée par l'image.

14. Procédé de commande d'un système médical (200, 300, 500, 600), dans lequel le système médical comprend : un support de sujet (206), dans lequel le support de sujet comprend une surface de support (208) destinée à recevoir un sujet (210) ; une première caméra (214) configurée pour imager une première partie de la surface de support ; et une seconde caméra (216) configurée pour imager une seconde partie de la surface de support, dans lequel la première partie et la seconde partie comprennent une région de chevauchement, dans lequel le support de sujet est mobile par rapport à la première caméra et à la seconde caméra le long d'un chemin prédéterminé (218) entre une première position (324), au moins une position intermédiaire (326) et une seconde position (328), dans lequel chacune des une ou plusieurs positions intermédiaires est située entre la première position et la seconde position ;
dans lequel le procédé comprend :
- la commande (400) de la première caméra pour acquérir des données d'image de première caméra initiales (342) descriptives d'un sujet sur le support de sujet lorsque le support de sujet est dans la première position ;
- la commande (402) de la seconde caméra pour acquérir des données d'image de seconde caméra initiales (344) descriptives du sujet sur le support de sujet lorsque le support de sujet est dans la première position ;
- la construction (404) d'une image tridimensionnelle initiale (346) du sujet en utilisant au moins une partie de la région de chevauchement des données d'image de première caméra initiales et des données d'image de seconde caméra initiales ;
- l'identification (406) en tant que première région obstruée (222) d'une région du sujet imagée par les données d'image de première caméra initiales mais obstruée dans les données d'image de seconde caméra initiales ; et
- l'identification (408) en tant que seconde région obstruée (224) d'une région du sujet imagée par les données d'image de seconde caméra initiales mais obstruée dans les données d'image de première caméra initiales ;
dans lequel l'exécution des instructions exécutables par machine amène en outre le système de calcul à réaliser ce qui suit au moins une fois :
- commander (410) le support de sujet pour qu'il se déplace vers l'une des une ou plusieurs positions intermédiaires ;
- commander (412) la première caméra pour acquérir des données d'image de première caméra supplémentaires (348) descriptives du sujet sur le support de sujet lorsque le support de sujet est dans ladite position des une ou plusieurs positions intermédiaires ;
- commander (414) la seconde caméra pour acquérir des données d'image de seconde caméra supplémentaires (350) descriptives du sujet sur le support de sujet lorsque le support de sujet est dans ladite position des une ou plusieurs positions intermédiaires ;
- construire (416) une image tridimensionnelle de première caméra (352) du sujet en utilisant les données d'image de première caméra initiales et les données d'image de première caméra supplémentaires ;
- construire (418) une image tridimensionnelle de seconde caméra (354) du sujet en utilisant les données d'image de seconde caméra initiales et les données d'image de seconde caméra supplémentaires ;
- construire (422) une image tridimensionnelle composite (358) du sujet en utilisant l'image tridimensionnelle initiale, l'image tridimensionnelle de première caméra et l'image tridimensionnelle de seconde caméra, dans lequel la première région obstruée est au moins partiellement remplacée par l'image tridimensionnelle de première caméra, et dans lequel la seconde région obstruée est au moins partiellement remplacée par l'image tridimensionnelle de seconde caméra.

15. Programme informatique comprenant des instructions exécutables par machine (340) destinées à être exécutées par un système de calcul (316) commandant un système médical (200, 300, 500, 600), dans lequel le système médical comprend : un support de sujet (206), dans lequel le support de sujet comprend une surface de support (208) destinée à recevoir un sujet (210) ; une première caméra (214) configurée pour imager une première partie de la surface de support ; et une seconde caméra (216) configurée pour imager une seconde partie de la surface de support, dans lequel la première partie et la seconde partie comprennent une région de chevauchement, dans lequel le support de sujet est mobile par rapport à la première caméra et à la seconde caméra le long d'un chemin prédéterminé (218) entre une première position (324), au moins une position intermédiaire (326) et une seconde position (328), dans lequel chacune des une ou plusieurs positions intermédiaires est située entre la première position et la seconde position ;
dans lequel l'exécution des instructions exécutables par machine amène le système de calcul à :
- commander (400) la première caméra pour acquérir des données d'image de première caméra initiales (342) descriptives d'un sujet sur le support de sujet lorsque le support de sujet est dans la première position ;
- commander (402) la seconde caméra pour acquérir des données d'image de seconde caméra initiales (344) descriptives du sujet sur le support de sujet lorsque le support de sujet est dans la première position ;
- construire (404) une image tridimensionnelle initiale (346) du sujet en utilisant au moins une partie de la région de chevauchement des données d'image de première caméra initiales et des données d'image de seconde caméra initiales ;
- identifier (406) en tant que première région obstruée (222) une région du sujet imagée par les données d'image de première caméra initiales mais obstruée dans les données d'image de seconde caméra initiales ; et
- identifier (408) en tant que seconde région obstruée (224) une région du sujet imagée par les données d'image de seconde caméra initiales mais obstruée dans les données d'image de première caméra initiales ;
dans lequel l'exécution des instructions exécutables par machine amène en outre le système de calcul à réaliser ce qui suit au moins une fois :
- commander (410) le support de sujet pour qu'il se déplace vers l'une des une ou plusieurs positions intermédiaires ;
- commander (412) la première caméra pour acquérir des données d'image de première caméra supplémentaires (348) descriptives du sujet sur le support de sujet lorsque le support de sujet est dans ladite position des une ou plusieurs positions intermédiaires ;
- commander (414) la seconde caméra pour acquérir des données d'image de seconde caméra supplémentaires (350) descriptives du sujet sur le support de sujet lorsque le support de sujet est dans ladite position des une ou plusieurs positions intermédiaires ;
- construire (416) une image tridimensionnelle de première caméra (352) du sujet en utilisant les données d'image de première caméra initiales et les données d'image de première caméra supplémentaires ;
- construire (418) une image tridimensionnelle de seconde caméra (354) du sujet en utilisant les données d'image de seconde caméra initiales et les données d'image de seconde caméra supplémentaires ;
- construire (422) une image tridimensionnelle composite (358) du sujet en utilisant l'image tridimensionnelle initiale, l'image tridimensionnelle de première caméra et l'image tridimensionnelle de seconde caméra, dans lequel la première région obstruée est au moins partiellement remplacée par l'image tridimensionnelle de première caméra, et dans lequel la seconde région obstruée est au moins partiellement remplacée par l'image tridimensionnelle de seconde caméra.
